# EUROPEAN PATENT APPLICATION

(11) **EP 1 548 100 A1**
(43) Date of publication of application: **29.06.2005**
(21) Application number: 03078549.7
(22) Date of filing: 11.11.2003
(51) Int. Cl.: C12N 5/08

(54) **Inhibition of stem cell differentiation, enhancement of proliferation and selective induction of apoptosis by Wnt factors**

(71) Applicant: DeltaCell B.V., 2333 CK Leiden (NL)
(72) Inventor: Kielman, Menno Frederik, 2251 RR Voorschoten (NL)
(74) Representative: Prins, Adrianus Willem, Mr. Ir.

(57) **Abstract**

The invention relates to a method for at least in part inhibiting differentiation of stem cells in a population of mammalian cells comprising upregulating a Wnt signaling pathway to a differentiation inhibiting level in said population of cells. The invention increases at the same time the number of stem cells in a population of mammalian cells compared to a reference population, and induces at least in part apoptosis in mesenchymal cell in a population of mammalian cells The invention also discloses a method for selective differentiation of a stem cell, comprising controlling the level of Wnt pathway activation. The invention is used for the proliferation and subsequent differentiation of embryonic stem cells and lung stem cells, and for *ex vivo* lung explant cultivation.

## Description

The invention relates to the differentiation of stem cells, in particular to organ tissue regeneration and recovery and to transplantation. The invention more in particular relates to the inhibition of stem cell differentiation by manipulating the Wnt pathway. More in particular, the invention relates to enhancing proliferation and differentiation of lung cells by manipulating the Wnt pathway.

Vital organs like the lung, the kidney, the liver, the pancreas, and the skin are characterized by the presence of organ-specific epithelial cells based on an organ specific matrix of connective tissue (mesenchymal) cells. The combination of said differentiated cells with the connective tissue matrix is related to the specific function of each such organ. These functions may be as varied as for example gas exchange in the lung, filtration in the kidney, detoxification and conjugation in the liver, insulin production in the pancreatic islet cells or protection against an hazardous environment by the skin. Disease or degeneration of such an organ is often life threatening, because degenerated or lost organ structure is often poorly replaced and because the specialized cells of one organ cannot take over the function of another organ.

Worldwide, solutions have been sought for the treatment of degenerative disease of organs and the skin and its appendices. For the skin, as a relatively simple organ structure, some advances have been made by application of autologous or heterologous skin transplants. Pieces of skin are treated (meshed) in order to be stretched out beyond their former size and cover the skin defects of a patient with meshed skin. Such transplants offer a basis for cells to proliferate and close the skin lesions.

For the specialized organs in the body, regenerating cells within the organ in situ would be most optimal because transplantation is often more difficult and less successful. Transplantation often leads to adverse effects of host versus graft or even graft versus host. It would also be a major achievement if organs or part thereof, could be kept outside of the body (syn. *ex vivo*) for a period of time, to give them a chance to recover by upregulating the proliferation and differentiation of the organ-specific cells. While the organ is *ex vivo*, the patient may be dependent of machines to take over the organ's function. In a later stage, said organ is placed back in the patient. This method would also be very suitable for bone marrow cells that can be taken out of the patient before treatment of leukemia, and be replaced after the treatment. Said bone marrow cells often are separated in various subpopulations and tested for the absence of leukemic cells to avoid the leukemic cells from reentering the host. Many cells do not survive this *ex vivo* handling; with the result that re-implantation of the bone marrow is less successful.

Especially such highly differentiated cells like for example the kidney cells, the insulin producing cells in the Islets of Langerhans of the pancreas, and glandular and/or hair follicle cells of the dermis, are also very difficult to recover, if possible at all, and very difficult to maintain, once taken out of their context in the body.

Many researchers try to find ways of extending the survival time of said specialized cells in culture, and possibilities to proliferate the cells whilst retaining their differentiated state. In practice, it seems to be hard to reach this goal. In the present invention this objective is reached by enhancement of the Wnt signaling pathway in stem cell/progenitor cells of such an organ comprising differentiated cells. As an example the extended survival time and the proliferation and differentiation of stem cells of the lung is disclosed.

One of the main potential applications of stem cells is their use in transplantation studies in which these cells reconstitute the cells in the diseased organ. Transplantation is preferably performed with stem cells that can either differentiate in vivo to the desired cell-type or with in vitro pre-differentiated stem cells. This pre-differentiation of stem cells *in vitro* to the desired cell-type is a process that was difficult to control before this invention.

The present invention describes a method to direct the differentiation of stem cells to a desired cell-type *in vitro* in order to use the differentiated cells for transplantation purposes (example IV). Control of this differentiation is achieved by limiting the enhancement of the Wnt signaling pathway to a certain window in these stem cells. Control of the Wnt-signaling pathway is achieved by the addition of components of the Wnt signaling pathway or affectors of gene expression that can interfere with the Wnt-signaling pathway. Without being thought by theory, it is thought that the fluctuation of the concentration of intracellular beta catenin is kept within a narrow range thereby promoting the differentiation of a limited number of cell-types.

The Wnt gene family encodes developmentally important secreted factors, involved in cell growth, differentiation and organogenesis (Wodartz & Nusse, 1998). Wnt genes encode a family of secreted glycoproteins that modulate cell fate and behavior in embryos through activation of receptor-mediated signaling pathways. Wnt signaling events are initiated by receptor activation involving binding to the cysteine-rich domain (CRD) of frizzled 7-transmembrane receptor protein (Fz) (Bhanot et al., 1996). A classical Wnt signal suppresses the activity of glycogen synthase kinase 3 (GSK-3), leading to changes in phosphorylation and increased stability of the β-catenin protein in the cytoplasm (Hinck et al., 1994). β-catenin is essential for activating target genes in response to Wnt signaling (Miller & Moon, 1996; Willert & Nusse, 1998), since it complexes with HMG box transcription factors of the TCF/LEF family (Behrens et al., 1996; Molenaar et al., 1996; Huber et al., 1996). Wnt sequences, patterns of expression and activities are highly conserved in evolution, so it has been possible to gain insights into the functions, and mechanisms of action, of the Wnt genes through a synthesis of genetic and cell biological approaches in different organisms. These studies suggest that there are functionally distinct WNT proteins as assayed by the ability to transform cells and by differences in embryonic responses to ectopic WNT signals. Moreover, gain-of-function and loss-of-function studies both support the involvement of Wnt proteins in modulating cell fate and cell behavior during vertebrate development, often through combinatorial interactions with other signaling pathways to regulate gene expression ( Moon RT, Brown JD, Torres M. Trends Genet 1997 Apr; 13(4): 157-62). This is supported by data on the ability and sensitivity of mouse embryonic cells to differentiate into three germ layers, which was inhibited by specific mutations in the adenomatous polyposis coli gene.(Kielman et al., 2002)

Components of the Wnt signaling pathway have been found to be present during organogenesis in the mouse (Roelink & Nusse, 1991; Buhler et al., 1993; Parr et al., 1993; Christiansen et al., 1995; Wang & Shackleford, 1996; Cho & Dressler, 1998; Korinek et al., 1998; Leimester et al., 1998). Moreover, loss of function of Wnt and Wnt-related genes leads to abnormal development in the mouse (McMahon & Bradley, 1990; Monkley et al., 1993; Takada et al., 1994; Stark et al., 1994; Galceran et al., 1999; Liu et al., 1999; Yamaguchi et al., 1999; Brisken et al., 2000; Lee et al., 2000). Wnt signaling is inhibited by the presence of Dickkopf proteins. Said Dickkopf (Dkk) proteins bind to the LRP co-receptor for Wnt. Modulating the Wnt pathway in cell culture can be performed by genetic modification (Kielman et al., 2002) or by administering soluble factors influencing the Wnt pathway. The latter method is used for monolayers of cells in cell culture wherein the cells had no tissue connection to each other like for example hemopoietic stem cells (Matthews et al., 2000)..In organ culture, the effects of soluble factors is not easily predicted or understood, because of the three dimensional structure of the organ and because the microenvironment of the cells is more complex by the presence of other cells such as connective tissue and blood vessels.

This invention describes the controlling of the canonical Wnt signaling pathway, and discloses as an example Wnt3a functioning as a protein of the canonical Wnt-pathway. The present invention demonstrates that Wnt3a inhibits the proteolytic breakdown of intra-cellular beta-catenin, thereby inhibiting the differentiation of pluripotent stem cells in the lung and of omni-potent stem cells.

In describing the present invention, the following terms will be employed, and are intended to be defined as indicated below.

Lung tissue in the present invention comprises at least two or more of the following compartments: the alveolar compartment, the bronchiolar compartment, the bronchial compartment and the tracheal compartment. A lung cell is defined herein as an epithelial cell of one of these four compartments. A population of lung cells comprises at least two lung cells. In this invention, in a preferred embodiment, a population of lung cells together functions in the exchange of gasses between the organism's blood compartment and the atmosphere. In the lung, epithelial cells and supporting tissue is present. The supporting tissue comprises connective tissue, nerves and lymph and blood vessels. In this application, connective tissue is described by the term mesenchym.

Omnipotent stem cells are cells that possess the ability to proliferate indefinitively and posses the capacity to differentiate into any possible cell-type that is present in the full-grown organism.

Pluripotent stem cell are cells that possess the ability to proliferate indefinitively and posses the capacity to differentiate into a large but limited number of cell-types that in general belong to a certain tissue such as the lung or a certain cell lineage such as the endoderm, ectoderm or mesoderm.

Stem cells can be genetically modified or not or they can be obtained by nuclear transfer or not.

The canonical Wnt-signaling pathway is described as the signaling cascade in which a collection of proteins controls the proteolytic breakdown of beta-catenin. The proteins in this pathway can be chemically modified by other proteins or may induce other molecules such as sugars and lipids that also can have an influence on the proteolytic breakdown of beta-catenin.

Wnt3a protein is part of a large family of proteins that have a close resemblance to each other in structure and function. Wnt proteins are strongly conserved during evolution in structure and function and are interchangeable between species. The invention therefore encloses all Wnt proteins or a functional part, derivative or analogue thereof that, like Wnt3a, can induce the canonical Wnt pathway. Other components of the Wnt signaling pathway or fragments of these components, or compounds mimicking the function of these components that in the end reduce the proteolytic breakdown of beta-catenin will have the same effect as Wnt3a and are also included in this patent application.

The terms "Wnt" or "*Wnt* gene product" or "*Wnt* polypeptide" when used herein encompass native sequence *Wnt* polypeptides, *Wnt* polypeptide variants, *Wnt* polypeptide fragments and chimaeric *Wnt* polypeptides. Optionally, the *Wnt* polypeptide is not associated with native glycosylation or palmitolyation. "Native glycosylation" refers to the carbohydrate moieties that are covalently attached to *Wnt* polypeptide when it is produced in the metazoan cell from which it is derived in nature. Native palmitolyation in its turn refers to the covalent attachment of lipid derivatives to the Wnt polypeptide when it is produced in the metazoan cell which is derived in nature (Willert et al, 2003). Accordingly, a human *Wnt* polypeptide produced in a non-metazoan cell is an example of a Wnt that is "not associated with native glycosylation" or palmitolyation. Sometimes, the *Wnt* polypeptide is unglycosylated or unpalmitolyated (e.g., as a result of being produced recombinantly in a prokaryote).

A "native sequence" polypeptide is one that has the same amino acid sequence as a polypeptide (e.g., *Wnt* polypeptide) derived from nature. Such native sequence polypeptides can be isolated from nature or can be produced by recombinant or synthetic means. Thus, a native sequence polypeptide can have the amino acid sequence of naturally occurring human polypeptide, murine polypeptide, or polypeptide from any other mammalian species.

The term "native sequence *Wnt* polypeptide" includes those *Wnt* polypeptides from any animal species (e.g., human, murine, rabbit, cat, cow, sheep, chicken, porcine, equine, etc.) as occurring in nature. The term "native sequence *Wnt* protein" includes the native proteins with or without the initiating N-terminal methionine (Met), and with or without the native signal sequence. The native sequence human and murine *Wnt* polypeptides known in the art are from about 348 to about 389 amino acids long in their unprocessed form reflecting variability (particularly at the poorly conserved amino-terminus and several internal sites), contain 21 conserved cysteines, and have the features of a secreted protein (see, e.g., *Wnt* polypeptides as in Gavin et al., supra; Lee et al., supra; Christiansen et al., supra; PCT/US94/14708 [WO 95/17416]). The molecular weight of a *Wnt* polypeptide is about 38-42 kD in a monomeric form.

A " functional part, derivative or analogue" of a Wnt polypeptide means a biologically active polypeptide as defined below having less than 100% sequence identity with a native sequence Wnt polypeptide. Such functional parts, derivatives or analogues include polypeptides wherein one or more amino acid residues are added at the N- or C-terminus of, or within, the native sequence; from about one to forty amino acid residues are deleted, and optionally substituted by one or more amino acid residues; and derivatives of the above polypeptides, wherein an amino acid residue has been covalently modified so that the resulting product has a non-naturally occurring amino acid. Ordinarily, a biologically active *Wnt* variant will have an amino acid sequence having at least about 90% amino acid sequence identity with a native sequence *Wnt* polypeptide, preferably at least about 95%, more preferably at least about 99%. A functional part means having an effector function that is directly or indirectly caused or performed by native sequence *Wnt* polypeptide, such as *Wnt*-3a. Effector functions of native sequence *Wnt* polypeptides include inhibition of differentiation and/or enhancement of proliferation and/or induction of apoptosis.

"Functional part or derivatives" include, but are not limited to, fragments of a native sequence and derivatives of a native sequence *Wnt* polypeptide and its fragments, provided that they have a biological activity in common with a corresponding native sequence *Wnt* polypeptide. The term "derivative" encompasses both amino acid sequence variants of *Wnt* polypeptide and covalent modifications thereof.

"Isolated" *Wnt* polypeptide has been purified from a Wnt source or has been prepared by recombinant or synthetic methods and is sufficiently free of other peptides or proteins (1) to show homology for at least 15 and preferably 20 amino acid residues of the N-terminal or of an internal amino acid sequence, or (2) to homogeneity by SDS-PAGE under non-reducing or reducing conditions using Coomassie blue or, preferably, silver stain.

The term "antibody" is used in the broadest sense and specifically covers monoclonal antibodies, antibody compositions with poly-epitope specificity, bispecific antibodies, diabodies, and single-chain molecules, as well as antibody fragments (e.g., Fab, F(ab').sub.2, and Fv), so long as they exhibit the desired biological activity.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohleret al., Nature 256:495 [1975], or may be made by recombinant DNA methods (see, e.g., U.S. Pat. No. 4,816,567 (Cabilly et al.)). The "monoclonal antibodies" may also be isolated from phage antibody libraries using the techniques in Clackson et al., Nature 352:624-628 [1991] and Marks et al., J. Mol. Biol. 222:581-597 [1991], for example.

The monoclonal antibodies herein specifically include "chimaeric" antibodies (immunoglobulins) in which a portion of the heavy and/or light chain is identical with or homologous to corresponding sequences in antibodies derived from a particular species or belonging with or homologous to corresponding sequences in antibodies derived from another species or belonging to another antibody class or subclass, as well as fragments of such antibodies, so long as they exhibited desired biological activity (Cabilly et al., supra; Morrison et al., Proc. Natl. Acad. Sci. USA, 81:6851-6855 [1984]).

The phrase "enhancement of proliferation of a cell" encompasses the step of increasing the extent of growth and/or reproduction of the cell relative to an untreated cell either *in vitro* or *in vivo*. An increase in cell proliferation in cell culture can be detected by counting the number of cells before and after exposure to a molecule of interest. The extent of proliferation can be quantified via microscopic examination of the degree of confluency. Cell proliferation can also be quantified using a thymidine or BrdU incorporation assay.

By "controlling differentiation of a cell" is meant the act of increasing the extent of the acquisition or possession of one or more characteristics or functions which differ from that of the original cell (i.e., cell specialization). This can be detected by screening for a change in the phenotype of the cell (e.g., identifying morphological changes in the cell and/or surface markers on the cell).

A "lung stem/progenitor cell" or "primitive lung cell" is one, which is able to differentiate to form a more committed or mature lung cell type.

"Mammal" for purposes of treatment refers to a human or non-human mammal, including, a domestic and farm animal, and a zoo, a sports, or a pet animal, such as a dog, a horse, a cat, a cow, etc. Preferably, the mammal is human.

In one embodiment, the present invention provides the use of a factor that enhances the canonical Wnt-signaling pathway to levels that prevent or inhibit or at least retard the differentiation of stem cells from the lung and of omni-potent stem cells and enhance their proliferation.

The concentration of factor needed to enhance the Wnt-signaling pathway in order to inhibit differentiation and to enhance proliferation, is depending on the stem cell type and the relevant factor. As an example, this application discloses the effective concentration needed for the proliferation of lung cells, but with the method of the invention, a skilled person is taught how to find the effective concentration for other cell types and organ types. For recombinant human Wnt3a as produced by R&D systems (cat nr. 1323-WN) the effective concentration for lung stem cells is between 20 and 2000 ng/ml. Therefore, this application discloses a method for at least in part inhibiting differentiation of stem cells in a population of mammalian cells comprising upregulating a Wnt signaling pathway to a differentiation inhibiting level in said population of cells.

Nowadays it is possible by differential adhesion and percoll gradient centrifugation techniques to isolate a stem cell population from lung and airway tissue. Long-term culture (for example for more than 4 or 5 days) of these stem cells without loosing the stem cell properties was not possible before this invention.

The present invention provides a method to expand the total number of somatic stem cells present in lung and airway tissue (that can include the tracheal compartment, the bronchial compartment, the bronchiolar compartment and the alveolar compartment) and of omni-potent stem cells (such as embryonic stem cells) *in-vitro,* whilst at least in part preserving their original stem cell properties. The application discloses a method for increasing the number of stem cells in a population of mammalian cells compared to a reference population, comprising upregulating a Wnt signaling pathway in said population of cells to a differentiation inhibiting level, wherein said reference population is not provided with said upregulating of a Wnt signaling pathway.

In one embodiment of this invention stem cells can be isolated from healthy lung tissue and expanded *in vitro* and be used as a source of stem cells that can be transplanted directly to a patient in order to generate new lung or airway tissue.

The invention now also enables a skilled person to isolate lung or airway stem cells from a lung sample of the patient's lung or airway tissue and to expand these patient-derived stem cell in sufficient numbers *in vitro* in order to return these stem cells to the patient. Therefore, this application discloses a method for selective differentiation of a stem cell, comprising controlling the level of Wnt pathway activation.

In another embodiment, the invention is related to genetic modification of the cells of a person with a genetic defect in the lung cells, and it teaches how to isolate, culture and expand the patient's own stem cells *in vitro* and to modify them genetically in order to correct the effect of this genetic defect present in the patient's germ line and to return these genetically modified stem cells to the patient. Therefore, the present invention teaches a cell, obtained by the method for selective differentiation comprising controlling a Wnt signaling pathway.

In another embodiment, because with the present invention stem cells can be cultured for a longer period of time, cultured stem cells from lung and airway tissue can be used as a source to generate differentiated lung and airway tissue *in vitro* in order to transplant these to a patient with a lung or airway defect. Therefore, in another embodiment, this application discloses a method for repopulating cells in a mammal comprising administering to said mammal a therapeutically effective amount of a stem cell or a lung stem cell as described before. This method is particularly useful for the treatment of people suffering from or susceptible to lung failure, said lung failure for example caused by lung surfactant-deficiency, emphysema, chronic respiratory distress, and/or cancer. The cells may be taken out of the body to be treated according to the present invention, or one may choose to treat the cells in the body. Therefore, the present invention discloses a method for at least in part inhibiting differentiation of stem cells, and/or increasing the numbers of stem cells, and/or enhancing the apoptosis of mesenchymal cells, and/or differentiating the stem cells in the mammalian body, preferably in a human body. In another embodiment the invention provides a method to prevent or retard the differentiation of omni-potent stem cells. Such omni-potent stem cells have the ability to differentiate into all the possible cell-types and cell-lineages that are present in the full-grown organism. Omni-potent stem cells are preferably isolated from the inner-cell mass of early embryos at the blastocyst stage. The invention is also valid for omni-potent stem cells that can be isolated from early embryos at other developmental stages as the blastocyst stage. The invention is also valid for omni-potent stem cells, that can be isolated from the genital ridge, the so called primordial germ cells (PGCs), of early embryos that already underwent the formation of the three germ layers, ectoderm, endoderm and mesoderm during gastrulation. The invention is also applicable to omni-potent stem cells that are obtained by the transfer of nuclei from differentiated somatic cells (preferably from a patient) to one of the omni-potent stem cells described in this patent.

One of the applications in which omni-potent stem cells are used is the generation of transgenic animals by transfecting the omni-potent cell with a DNA-construct in such a way that the original DNA content of that cell is changed. Such a DNA construct can be maintained in the cell by an interchromosomal integration (nuclear or mitochondrial) or as an episomal (extra-nuclear) DNA molecule. Maintenance of the omni-potent cells can be facilitated by the addition of leukocyte inhibitory factor or other growth factors added in a purified form or produced by a cell-line. This invention provides an alternative to these growth factors as its addition at an effective concentration helps to maintain the omni-potent characteristics of the stem cells.

Another embodiment of this invention is its use in the generation and isolation of omni-potent stem cells from the embryo. Omni-potent stem cells can be isolated from the embryo at different developmental stages. The difficulty in this process is the loss of the omni-potent properties of the stem cells due to differentiation into various cell-types. This invention prevents or retards the differentiation of these omni-potent stem cells thereby facilitating the isolation of omni-potent stem cell lines from embryos whilst maintaining their omni-potent properties. Isolation of omni potent stem cells from early embryos is without this invention not possible or very difficult in many species including humans. This invention makes it now possible to isolate with a much higher frequency and efficiency than previously was possible, omni-potent stem cells from various mammalian species including humans.

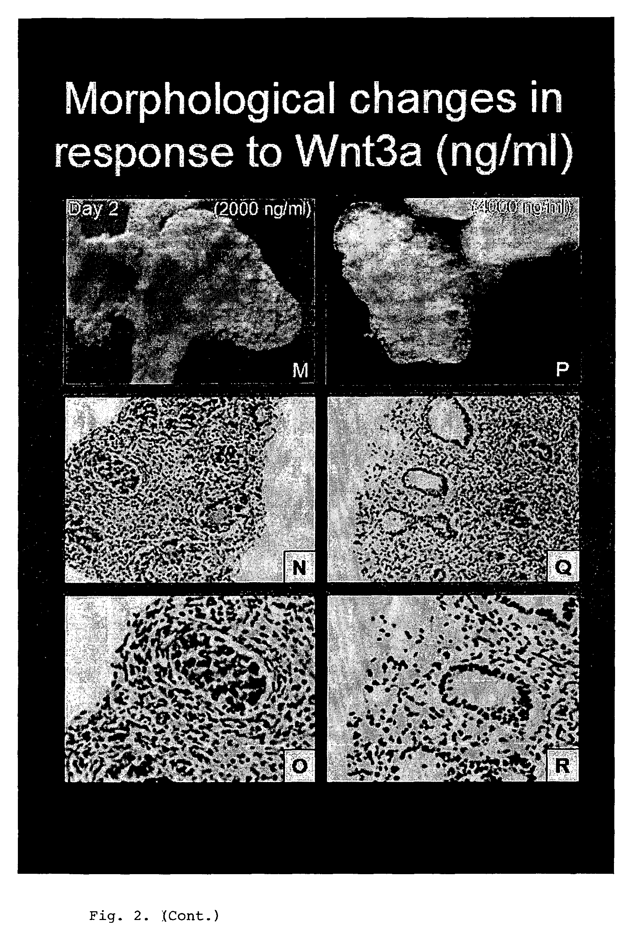

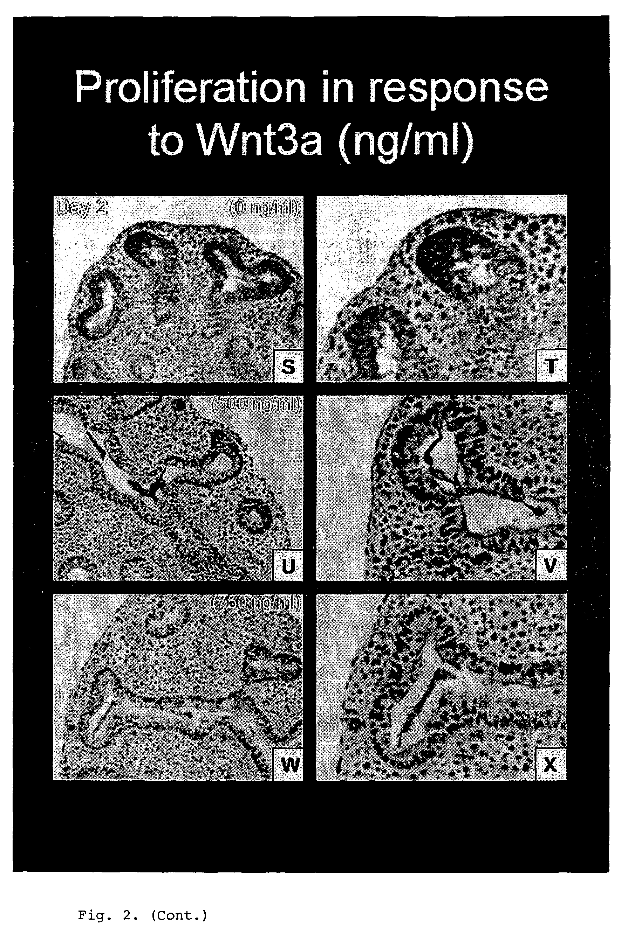

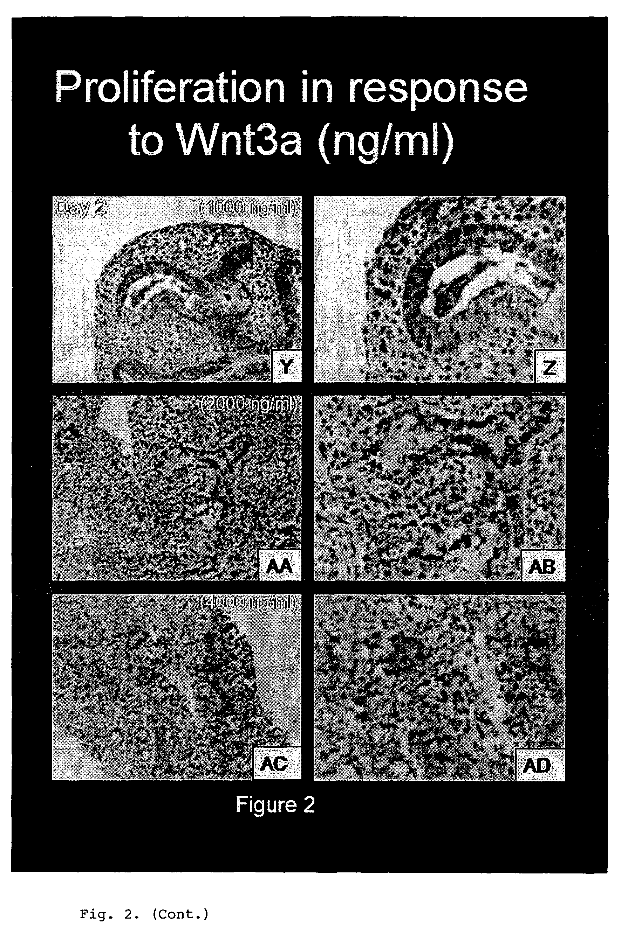

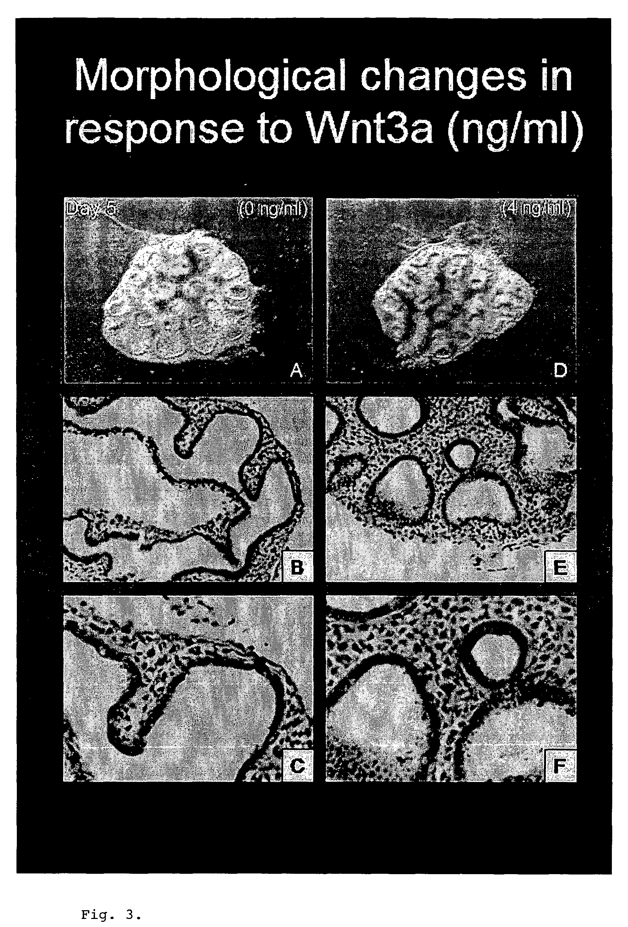

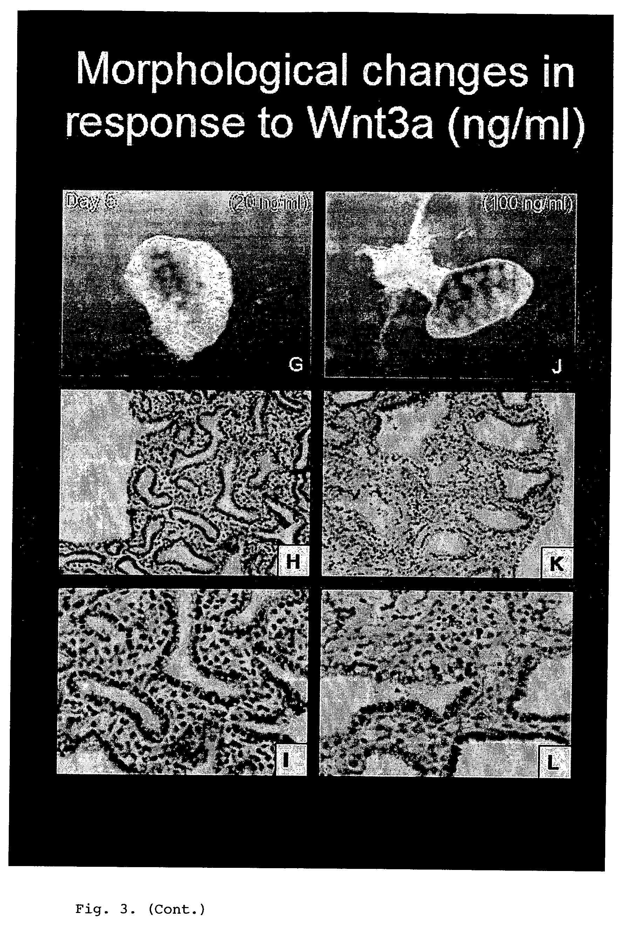

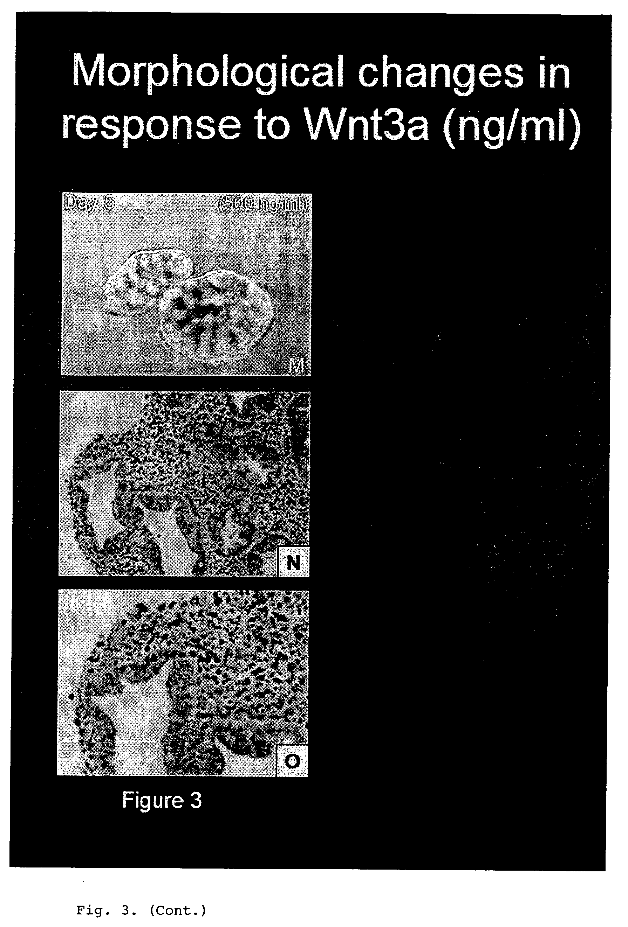

In another embodiment, this application teaches that the induction of apoptosis by activation of the Wnt pathway is possible in every cell type present in the lung, i.e. terminally differentiated cells as well as stem cells. The concentration of factor needed to induce apoptosis in a specific cell-type is dependent on the factor itself and on the cell-type. This concentration is for cell-types present in the lung depicted in example 1, figure 2 and 3 The level of Wnt polypeptide needed for the apoptosis of mesenchymal cells is close to the level needed for inhibition of the differentiation and the enhancement of stem cell proliferation. Therefore, the present application discloses a method for inducing apoptosis in mesenchymal cell in a population of mammalian cells comprising upregulating a Wnt signaling pathway in said population of cells to a differentiation inhibiting level. Preferably, said population also comprises a stem cell.

In another aspect, this apoptosis of mesenchymal cells prevents overgrowth of the somatic stem cells during their culture *in vitro* thereby facilitating the purification and selective culture of lung stem cells *in vitro.*

Differentiation of omni- or pluri-potent stem cells is dependent on the strength of the Wnt signaling pathway in these cells. Stem cells in which the Wnt signaling pathway is high are limited in their differentiation capacity by the action of the Wnt signal. A stem cell in which the possible differentiating capacity or amplitude of the Wnt signaling pathway is maximal can differentiate to all its derivatives. A stem cells in which the amplitude of the Wnt-signaling pathway is limited to a certain window is limited in its differentiation capacity and is restricted to differentiate to certain cell-types.

This invention provides a method to direct the differentiation of omnipotent and pluripotent stem cells to a limited number of desired cell types such as epithelial alveolar type-II cells by restricting the Wnt signal strength to a certain window. The present invention further discloses a differentiated cell, obtained by a method for selective differentiation of a stem cell, comprising controlling the level of Wnt pathway activation. Said controlling of the Wnt pathway is achieved.

This invention further discloses a soluble compound useful for the propagation or the selected differentiation of a stem cell. Such a compound may be included in a cell culture medium. Therefore, the present invention provides a kit containing a cell culture medium and a component that can influence the Wnt signaling pathway. This kit may of course be used for several purposes like the proliferation of stem cells, or the differentiation of stem cells and it may also be useful fro the cultivation and differentiation of other cells than stem cells.

### EXAMPLES

### Example 1

Generation of new lung tissue ex vivo

### Preservation and potential growth of alveolar tissue in a murine lung by in culture treatment with recombinant Wnt3a polypeptide.

To provide proof of evidence, an in vitro explant lung culture (obtained from a mouse) is used. Generation of new alveolar tissue in patients can be stimulated by activation or re-activation of alveolar bud formation. Growth and branching of alveolar buds, followed by transformation into alveolar ducts, sacs and pouches, results in the establishment of pulmonary acini.

This is a general differentiation principle in both the fetal and the adult mammalian

As a proof of evidence, it is therefore shown that treatment with selected molecules, involved in a Wnt-pathway, inhibits the differentiation process of alveolar differentiation. Inhibition of this differentiation process is achieved by applying a purified Wnt3a polypeptide to an in vitro lung explant culture of 13-day-old (E13) mouse embryos.

### Material& Methods

### In vitro lung explant cultures

The (fetal) murine lung explant culture was generated according to our standard protocol. Briefly, complete lungs or individually dissected lung lobes were isolated from fetal mice and cultured for 1 to 5 days in a hanging drop culture system. The Wnt3a polypeptide was administered to the culture medium in different concentrations, ranging from 0-4000 ng/ml. The effect of this treatment was monitored by stereomicroscopy. The lung explant cultures were subsequently fixed for 30 minutes in 10% paraformaldehyde (PFA), 30 minutes in 2% PFA and 30 minute sin 4% PFA. After fixation the lung explants were dehydrated through an increasing series of 2-isopropanol and subsequently embedded in paraffin. For the histological analysis 4um thick sections were cut and stained with Hematoxilin and Eosin using standard techniques.

For the immunohistochemical analysis comparable sections were used and treated with and anti-PCNA monoclonal antibody followed by a goat antimouse horse reddish peroxidase conjugate. Detection of the PCNA epitopes was carried out by treating the sections with di-aminobenzidine (DAB) for 15'.

### Materials:

As a source for Wnt3a was used, recombinant mouse Wnt-3a from R&D systems (catalog Nr: 1324-WN, R&D systems, Inc. Minneapolis USA). As culture medium was used DMEM/HAM's F12 (1:1) from Biochrom AG, catalog nr. FG 4815.

The antibody against PCNA was purchased from Zymed Laboratories Inc., (cat nr.18-0110). The secondary conjugate used was from Jackson Laboratories, Inc (catalog nr 115-056-062).

### Results:

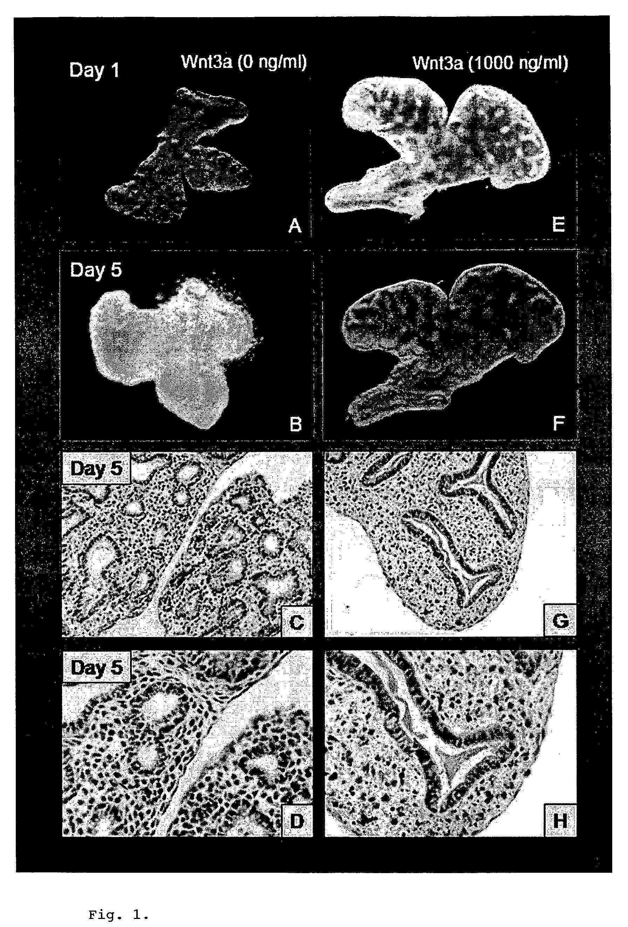

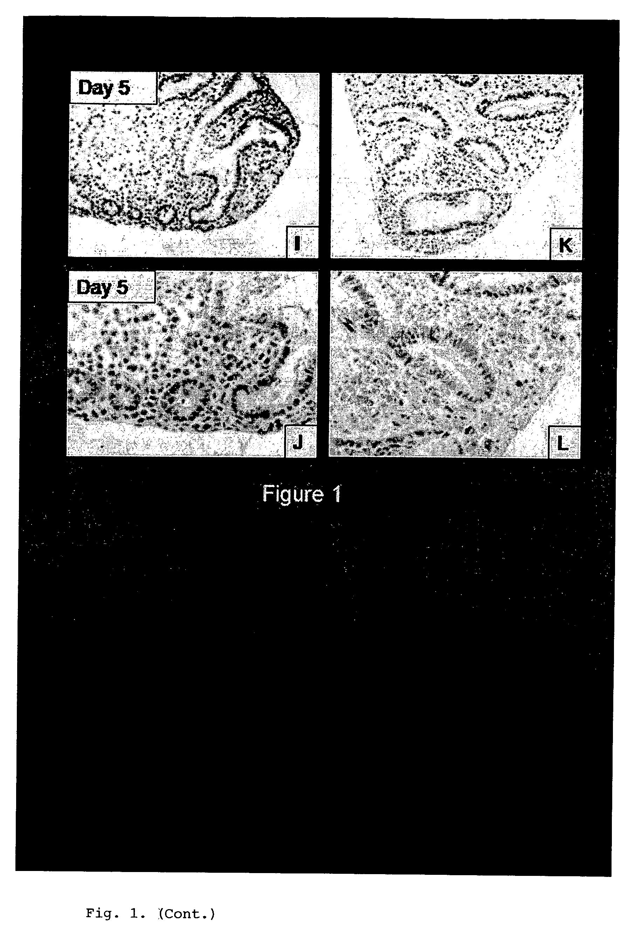

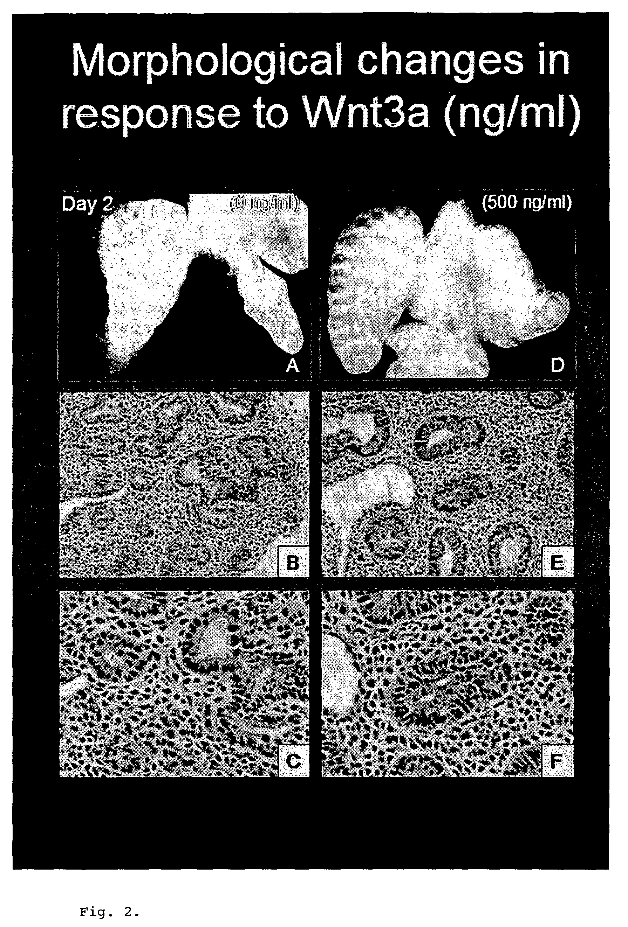

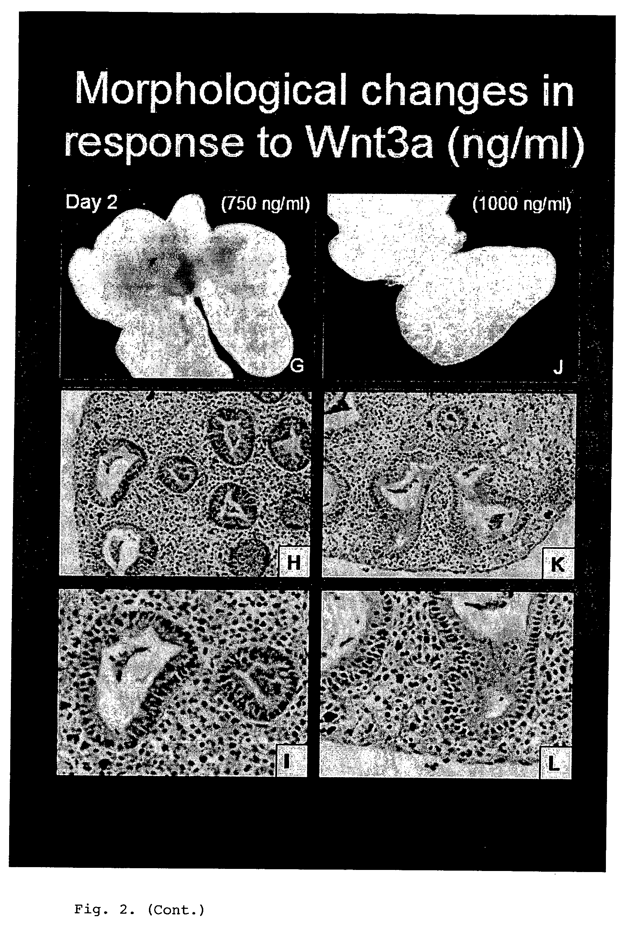

Treatment of the lung cultures of embryonic day 13 (E13) lung explants with 1000 ng/ml Wnt3a polypeptide resulted in a total block of lung differentiation after 16 hours (Fig. 1E). Even after five days culture under these conditions no significant differentiation and alveolar development could be observed by stereomicroscopy (Fig. 1F).

Histological analysis of these five day old cultures revealed that the differentiation of the primordial lung epithelium was arrested at a stage comparable to that of the start of the culture (E13). Furthermore, most of the mesenchymal cells surrounding the epithelium displayed picnotic nuclei, indicating that these cells had undergone apoptosis. This was in sharp contrast with the epithelium that contained no apoptotic cells (Fig. 1H).

To analyze the viability of the epithelium and the surrounding mesenchym, the sections were stained for the proliferation marker "Proliferating Cell Nuclear Antigen" (PCNA, Fig 1I-J). This immunohistochemical staining showed that the epithelium was still proliferating whereas most of the mesenchymal cells were inactive or dead (Fig. 1K+L). This in contrast to the untreated lung explant cultures were both the epithelium and the mesenchym were strongly proliferating (Fig 1I+J).

Above results indicate that the Wnt3a polypeptide has a strong inhibitory effect on the development and differentiation of the fetal lung.

In order to determine the concentration range in which the Wnt3a polypeptide is effective total lung explants were cultured in the presence of Wnt3a in a range of 0 ng/ml, 500 ng/ml, 750 ng/ml, 1000 ng/ml, 2000, ng/ml and 4000 ng/ml Fig. 2A-R).

Stereomicroscopic analysis showed already after one day a deleterious effect on lung development with the Wnt3a concentrations, 2000 ng/ml and 4000 ng/ml (Fig 2M+P).

After 2 days of culture, these lung explants were fixed and embedded in paraffin for immunohistological analysis.

The histological analysis of H/E stained sections showed that the mesenchymal cell population undergoes a rapid induction of apoptosis within the range 1000-4000 ng/ml Wnt3a. Apoptosis is also induced in the epithelium at a concentration of 2000-4000 ng/ml but is not detectable at a concentration of 1000 ng/ml. The mesenchym however, becomes at a concentration higher than 750 ng/ml clearly apoptotic indicating that the induction threshold for apoptosis is different for the epithelium and the mesenchym. At the concentration of 750 ng/ml apoptosis in the mesenchym is clearly detectable but not yet abundant, whereas the epithelium showed no sign of apoptosis whatsoever Fig. 2H+I).

Immunohistochemical staining of PCNA showed in addition a Wnt3a dependent proliferation upon the lung explant cultures. Without addition of Wnt3a the periphery of the lung containing the growing lung buds show a stronger staining for PCNA than the more central located bronchial epithelium, indicating that the periphery of the lung is stronger proliferating than the central part (Fig 2S+T). Addition of 500 ng/ml Wnt3a to the medium, however, shows a marked overall increase in the intensity and frequency of PCNA staining in the centrally located bronchial epithelium indicating that proliferation is enhanced by Wnt3a at a dosage of 500 ng/ml Wnt3a (Fig 2U+V). A further increase of the Wnt3a concentration to 750 ng/ml (Fig. 2W+X) does not lead to a further increase the intensity and frequency of the PCNA staining but instead is comparable to the control situation (Fig 2S+T). Addition of 1000 ng/ml Wnt3a reduces the PCNA staining below the control situation (Fig 2Y+Z), whereas at 2000 and 4000 ng/ml Wnt3a (Fig 2AA+AD), PCNA staining was completely negative indicating that proliferation was completely blocked, probably due to the apoptosis induced in the mesenchym and epithelium (Fig 2).

To more exactly determine the concentration of Wnt3a that is effective upon lung development, differentiation and apoptosis, fetal lung explants were cultured for a longer period (4 days) in the presence of lower concentrations of Wnt3a, i.e.; 0 ng/ml, 4ng/ml, 20ng/ml, 100ng/ml and 500ng/ml (Fig 3A-O).

None of these concentrations was effective in completely blocking the differentiation of fetal lung development, although differentiation at a concentration of 500 ng/ml (Fig 3N+O) was delayed as was displayed by the primordial morphology of the epithelium compared to the control situation (Fig 3B+C). Between 100-500 ng/ml the mesenchym showed an increasing level of apoptosis, whereas in the epithelium no sign of apoptosis could be detected (Fig. 3K+L and 3N+O).

Between 4 and 20 ng/ml no clear effects were visible at the level of apoptosis and differentiation compared to the control situation (Fig. 3B+C, 3E+F and 3H+I)).

### Conclusions

The addition of Wnt3a has a profound effect upon fetal lung development. Differentiation, cell-proliferation and apoptosis are greatly altered upon addition of Wnt3a to the culture medium. The effective concentration depends on the cell-type and the cellular function.

Differentiation of the primordial epithelium is effectively inhibited between a concentration of 20 and 2000 ng/ml with an optimum between 500 and 1000 ng/ml.

Proliferation of the bronchial and alveolar epithelium is markedly enhanced at 500 ng Wnt3a/ml culture medium, whereas at concentrations of above 2000ng/ml proliferation is inhibited.

Apoptosis of the mesenchym is induced at concentrations higher than 100 ng/ml, whereas apoptosis in the epithelium is induced at concentrations higher than 1000 ng/ml.

The effects of Wnt3a upon differentiation, proliferation and apoptosis are strongly dependent upon cell-type and dosage. Our results show that at certain concentrations Wnt3a can at the same time inhibit alveolar differentiation, enhance epithelial proliferation and induce apoptosis in mesenchymal cells. The range in which all these three events occur in the hanging drop culture system is between 100 and 1000 ng Wnt3a/ml culture medium. The addition of Wnt3a therefore can be used as a method to selectively culture and expand in total number the undifferentiated stem-cell population of the lung and to selectively induce apoptosis in a mesenchymal cell population.

### Example II

Ex-vivo culture of a purified population of Lung stem cell and maintenance of their pluripotent properties by addition of Wnt3a to the culture medium.

### Experimental set up:

A population of lung epithelial stem cells are isolated from mouse fetal lung explants of mice transgenic for a constitutive expressing fluorescent protein (Green Fluorescent protein (GFP) or DsRed, using differential adhesion, cell-sorting or micro dissection. These fluorescent stem cells are cultured and expanded in vitro using Wnt3a as a factor to inhibit their differentiation and to stimulate their proliferation. This stem cell population is first cultured for several passages in vitro and thereafter transplanted to an in-vitro lung explant culture of which the fetal lung is isolated from a non-fluorescent mouse strain in order to follow the fate of the fluorescent transplanted stem cells.

### Results:

The results show clearly that the transplanted fluorescent cell population is integrated into the recipient lung tissue. Marker analysis shows further that the stem-cell differentiates to the different cell-types present in the lung, such as alveolar type-I cells, bronchial cells, and alveolar type-II cells thereby indicating that the cultured stem cell population had maintained their pluripotent properties during their in vitro culture in the presence of Wnt3a.

### Example III

**Culture and maintenance of the pluripotent character of embryonic stem cells using addition of Wnt3a as a factor to inhibit their differentiation.**

### Experimental set up:

Embryonic stem cells from the mouse, constitutively expressing a LacZ, or GFP reporter gene, are cultured in the presence of Wnt3a as a factor to inhibit their differentiation. These stem cells are cultured and expanded in vitro for several passages and subsequently injected subcutaneously in a syngenic recipient mouse to allow the ES cells to differentiate into a teratoma.

### Results:

Immuno-histological analysis of these teratomas clearly shows the presence of different cell-types and tissues. Tissues that for example are detected are neural tissue, bone, muscle and lung tissue, indicating that the embryonic stem cells maintain their omnipotent properties during their in vitro culture with wnt3a.

### Example IV

Directing of differentiation of embryonic stem cells by controlling the strength of the Wnt signaling pathway during differentiation.

### Experimental set up:

Embryonic stem cells were transfected with a fluorescent marker specific for the alveolar type-II cell. This lung tissue specific marker is only expressed in the alveolar type-II cell and the bronchiolar clara cell and not in the embryonic stem cell itself.

### Results:

In vitro differentiation of the embryonic stem cells carrying this marker gene results in the differentiation of a small percentage alveolar type-II cells and clara cells as evidenced by their fluorescent expression. However, by the addition of Wnt3a at a specific dosage, as a factor to influence the differentiation process the number of fluorescent cell is significantly increased indicating that influencing the Wnt signaling pathway to a certain level can direct differentiation of embryonic stem cells to a lung tissue.

### Legends to Figures

Figure 1A-1L: Lung explant culture and subsequent immuno-histological analysis after five days, without the addition (fig 1A-D and 11+J) and with the addition of Wnt3a (1000 ng/ml, fig 1 E-H and 1 K+L). Note the arrest of alveolar differentiation , the primordial character of the epithelium and the massive induction of apoptosis in the mesenchym in the presence of Wnt3a five days after the start of the culture.
Figure 1I-L : Immunohistochemical analysis of employing PCNA as a marker for proliferation.
Figure 1 I+J; PCNA staining five days after the start of the culture without the addition of Wnt3a. Figure 1K+L; PCNA staining after five days of culture in the presence of Wnt3a (1000 ng/ml).
   Note the strong reduction in proliferation of the mesenchym versus the epithelium in the presence of Wnt3a.

Figure 2A-2R: Lung explant culture and subsequent histological analysis 2 days after the start of the culture in the presence of Wnt3a at a concentration of 0 ng/ml (2A-C); 500 ng/ml (2D-F); 750 ng/ml (2G-I); 1000 ng/ml (2J-L); 2000 ng/ml (2M-O) and 4000 ng/ml (2P-R).
   Note the increasing number of apoptotic cells in the mesenchym above 500 ng/ml Wnt3a and the induction of apoptosis in the epithelium above 2000 ng/ml Wnt3a.
Figure 2S-2AD.: Immunohistochemical analysis of employing PCNA as a marker for proliferation 2 days after the start of the culture in the presence of Wnt3a at a concentration of 0 ng/ml (2S+T); 500 ng/ml (2U+V); 750 ng/ml (2W+X); 1000 ng/ml (2Y+Z); 2000 ng/ml (2AA+AB) and 4000 ng/ml (2AC+AD). Note the elevated PCNA staining, compared to the control, in the bronchial epithelium in the prensence of 500 and 750 ng/ml Wnt3a and the absence of proliferation at concentrations above 2000 ng/ml.
Figure 3 A-F: Lung explant culture and subsequent histological analysis 4 days after the start of the culture in the presence of Wnt3a at a concentration of 0 ng/ml (3A-C); 4 ng/ml (3D-F); 20 ng/ml (3G-I); 100 ng/ml (3J-L); and 500 ng/ml (2M-O). Note the increasing number of apoptotic cells in the mesenchym between above 100 ng/ml Wnt3a and the undifferentiated character of the epithelium four days after culture in the presence of 500 ng/ml Wnt3a (2M-O).

### References

Behrens J, von Kries JP, Kuhl M, Bruhn L, Wedlich D, Grosschedl R and Birchmeier W. 1996. Functional interaction of beta-catenin with the transcription factor LEF-1. Nature, 382:638-42.
Bhanot P, Brink M, Samos CH, Hsieh JC, Wang Y, Macke JP, Andrew D, Cabilly et al :U.S. Pat. No. 4,816,567
Nathans J and Nusse R. 1996. A new member of the frizzled family from Drosophila functions as a Wingless receptor. Nature, 382:225-30.
Bienz M, Clevers H. Linking colorectal cancer to Wnt signaling. Cell. 2000 Oct 13;103(2):311-20.
Brown JD, Hallagan SE, McGrew LL, Miller JR and Moo RT. 2000. The maternal Xenopus beta-catenin signaling pathway, activated by frizzled homologs, induces goosecoid in a cell non-autonomous manner. Dev Growth Differ, 42:347-57, 2000.
Clackson T, Hoogenboom HR, Griffiths AD, Winter G. Making antibody fragments using phage display libraries. Nature. 1991 Aug 15;352(6336):624-8.
Hinck L, Nelson WJ and Papkoff J. 1994. Wnt-1 modulates cell-cell adhesion in mammalian cells by stabilizing beta-catenin binding to the cell adhesion protein cadherin. J Cell Biol, 124:729-41.
Kielman, M.F., Rindapää, M., Gaspar, C., van Poppel N., Breukel, C., van Leeuwen, S., Taketo, M.M., Roberts, S., Smits, R., Fodde, R. 2002. Apc modulates embryonic stem-cell differentiation by controlling the dosage of Beta catenin signaling. Nature Genetics 32:594-605
Kohler G, Milstein C. Continuous cultures of fused cells secreting antibody of predefined specificity. Nature. 1975 Aug 7;256(5517):495-7.
Korinek V, Barker N, Willert K, Molenaar M, Roose J, Wagenaar G, Markman M, Lamers W, Destree O and Clevers H. 1998. Two members of the Tcf family implicated in Wnt/beta-catenin signaling during embryogenesis in the mouse. Mol Cell Biol, 18:1248-56.
Lee SM, Tole S, Grove E and McMahon AP. 2000. A local Wnt-3a signal is required for development of the mammalian hippocampus. Development, 127:457-67.
Marks JD, Hoogenboom HR, Bonnert TP, McCafferty J, Griffiths AD, Winter G. By-passing immunization. Human antibodies from V-gene libraries displayed on phage. J Mol Biol. 1991 Dec 5;222(3):581-97.
McMahon AP, Gavin BJ, Parr B, Bradley A and McMahon JA.1992. The Wnt family of cell signalling molecules in postimplantation development of the mouse. Ciba Found Symp, 165:199-212; discussion 212-8.
Matthews, W., Austin, T.W.,1997 Uses of Wnt polypeptides US patent 6,159,462
Morrison SL, Johnson MJ, Herzenberg LA, Oi VT. Chimeric human antibody molecules: mouse antigen-binding domains with human constant region domains. Proc Natl Acad Sci USA. 1984 Nov;81(21):6851-6855.
Nusse R and Varmus HE. 1992. Wnt genes. Cell, 69:1073-87
Reya T., Duncan, A.W.; Ailles, L.; Domen, J.; Scherer, D.C.; Willert, K.; Hintz, L.; Nusse, R.; Weissmann, I.L.: A role for Wnt signalling in self-renewal of haematopoietic stem cells Nature, May 2003, 409-414
Stark K, Vainio S, Vassileva G and McMahon AP.1994. Epithelial transformation of metanephric mesenchyme in the developing kidney regulated by Wnt-4. Nature, 372:679-83.
Willert K and Nusse R. 1998. Beta-catenin: a key mediator of Wnt signaling. Curr Opin Genet Dev, 8:95-102.
Willert K, Brown JD, Danenberg E, Duncan AW, Weissman IL, Reya T, Yates JR 3rd, Nusse R. Wnt proteins are lipid-modified and can act as stem cell growth factors. Nature. 2003 May 22;423(6938):448-52.
Wodarz A and Nusse R. 1998. Mechanisms of Wnt signaling in development. Annu Rev Cell Dev Biol, 14:59-88.

## Claims

1. A method for at least in part inhibiting differentiation of stem cells in a population of mammalian cells comprising upregulating a Wnt signaling pathway to a differentiation inhibiting level in said population of cells.

2. A method for increasing the number of stem cells in a population of mammalian cells compared to a reference population, comprising upregulating a Wnt signaling pathway in said population of cells to a differentiation inhibiting level, relative to said signaling pathway in said reference population.

3. A method for inducing apoptosis in a mesenchymal cell in a population of mammalian cells comprising upregulating a Wnt signaling pathway in said population of cells to a differentiation inhibiting level.

4. A method for selective differentiation of a stem cell, comprising controlling the level of Wnt pathway activation.

5. A differentiated cell obtained by the method of claim 4.

6. A method of any of claims 1 to 3 wherein said population of cells is arranged in a three dimensional structure.

7. A method according to any of claims 1 to 4 wherein said upregulating and/or controlling comprises providing said population with a factor capable of upregulating a Wnt signaling pathway in said cells.

8. A method according to claim 7, wherein said factor comprises a soluble isolated Wnt-factor.

9. A method according to claim 7, wherein said factor comprises an antibody and/or a monoclonal antibody.

10. A method according to claim 7 wherein said factor comprises Wnt-3a or a functional part, derivative or analogue thereof.

11. A method according to claim 7 wherein said factor is present in an amount between 20 and 2000 nanogram/ml culture fluid.

12. A method according to claim 7 wherein said factor is present in an amount between 50 and 1000 nanogram/ml culture fluid

13. A method according to claim 7 wherein said factor is present in an amount between 500 and 1000 nanogram/ml culture fluid.

14. A method according to any of claims 1, 2 or 4, wherein said stem cell is a lung stem cell, or an embryonic stem cell.

15. A method according to any of claims 1 to 4, wherein said population of cells is *in vitro*.

16. The method of claim 1 or 2 wherein said population of cells is present in a mammal.

17. A method according to of claim 15 wherein said mammal is a human.

18. A method according to of claim 15 wherein said mammal is suffering from, or is susceptible to, lung failure.

19. A method according to of claim 17 wherein said lung failure is caused by lung surfactant-deficiency, emphysema, chronic respiratory distress, and/or cancer.

20. A stem cell or population comprising said stem cell obtained by a method according to any one of claims 1-4, and/or 6-19.

21. A stem cell or population comprising said stem cell according to claim 20 wherein said stem cell comprises a lung stem cell or an embryonic stem cell.

22. A method for providing a mammal with a stem cell comprising administering to said mammal a cell according to claim 20 or 21.

23. A method according to claim 22, wherein said stem cell comprises a lung cell.

24. A kit comprising a stem cell culture medium and a component that can influence the Wnt signaling pathway.
